# EUROPÄISCHE PATENTSCHRIFT

(11) **EP 1 893 166 B1**
(45) Veröffentlichungstag und Bekanntmachung des Hinweises auf die Patenterteilung: **16.01.2013**
(21) Anmeldenummer: 06754397.5
(22) Anmeldetag: 16.06.2006
(51) Int. Cl.: A61K 8/06, A61K 8/39, A61K 8/55, A61K 8/60, A61K 8/86, A61Q 5/06

(54) **MIKROEMULSION UND IHR GEBRAUCH**
MICROEMULSION AND USE THEREOF
MICROEMULSIONS ET LEUR UTILISATION

(30) Priorität: 21.06.2005 DE 102005028985
(43) Veröffentlichungstag der Anmeldung: 05.03.2008
(73) Patentinhaber: Henkel AG & Co. KGaA, 40589 Düsseldorf (DE)
(72) Erfinder: BARRELEIRO, Paula, 40724 Hilden (DE); HLOUCHA, Matthias, 50677 Köln (DE); DORN, Kerstin, 40724 Hilden (DE); SCHEFFLER, René, 25479 Ellerau (DE); HENTRICH, Dirk, 22457 Hamburg (DE); KNAPPE, Thorsten, 22869 Hamburg (DE)
(86) Internationale Anmeldenummer: PCT/EP2006/005788
(87) Internationale Veröffentlichungsnummer: WO 2006/136331

(56) Entgegenhaltungen:
- DE-A1- 10 213 956
- DE-A1- 19 813 057
- US-A1- 2001 036 450
- PATENT ABSTRACTS OF JAPAN Bd. 2003, Nr. 10, 8. Oktober 2003 (2003-10-08) & JP 2003 160448 A (MANDOM CORP), 3. Juni 2003 (2003-06-03)

## Beschreibung

Die vorliegende Erfindung betrifft bei Raumtemperatur hochviskose ölhaltige kosmetische oder pharmazeutische Zusammensetzungen in Form einer Mikroemulsion mit geringer Hautreizung und ihre Verwendung zur Modellierung von Haar.

Um kosmetische Mittel mit erwünschten Eigenschaften zu erhalten, müssen diesen in der Regel Emulgatoren zugesetzt werden. Die Emulgatoren ermöglichen es, Emulsionen aus hydrophoben und hydrophilen Bestandteilen zu stabilisieren. Zur Herstellung einer stabilen Emulsion müssen den Formulierungen hierbei in der Regel relativ hohe Konzentrationen an Emulgatoren zugesetzt werden.

So werden etwa in der EP1386601 Haarwachsprodukte mit flüssiger oder cremeförmiger Konsistenz beschrieben, die bis zu mehr als 20 Gew.-% Emulgatoren enthalten.

Ein Problem hierbei besteht darin, dass es bei Verwendung dieser Produkte aufgrund der hohen Konzentration an Emulgatoren, insbesondere bei Verwendung kurzkettiger Emulgatoren, zur Hautreizung kommen kann.

Aufgabe der vorliegenden Erfindung war es daher kosmetische Mittel wachsartiger Konsistenz, insbesondere ein Gelwachs, zur Verfügung zu stellen, die zu geringeren Hautirritationen führen, als herkömmlicherweise verwendete kosmetische Mittel wachsartiger Konsistenz.

Überraschenderweise wurde nun festgestellt, dass beim Einsatz von Molekülen, die eine hydrophile Kette umfassen, die beidseitig hydrophobe Köpfe aufweist, insbesondere beim Einsatz von beidseitig hydrophob modifizierten Polyolen, die Menge an Emulgatoren deutlich gesenkt werden kann, und dadurch das Problem von Hautirritationen verhindert oder zumindest deutlich vermindert werden kann.

Die Verwendung solcher Moleküle als Ersatzstoffe für Tenside in bei Raumtemperatur flüssigen kosmetischen Zusammensetzungen wurde bereits in der Patentschrift EP 0814753 B1 und in der Offenlegungsschrift EP 1120101 A2 beschrieben. EP 0814753 B1 betrifft hierbei kosmetische oder dermatologische Gele auf der Basis von Mikroemulsionen, während in EP 1120101 A2 Nanoemulsionen beschrieben werden. Bei Raumtemperatur hochviskose, wachsartige Zusammensetzungen, die solche Moleküle enthalten, sind im Stand der Technik jedoch noch nicht bekannt.

Ein erster Gegenstand der vorliegenden Erfindung sind daher kosmetische oder pharmazeutische Zusammensetzungen in Form einer Mikroemulsion, enthaltend
(a) mindestens einen Diester aus PEG 100-500 und einer C₁₀₋₂₆-Carbonsäure in einer Menge von 0,5-4 Gew.-%, insbesondere 1-3 Gew.-%, als Molekül, das eine hydrophile Kette umfasst, die beidseitig hydrophobe Köpfe aufweist,
(b) mindestens einen Emulgator in einer Menge von 5-25 Gew.-%, wobei mindestens ein nichtionischer Emulgator ausgewählt aus der Gruppe der ethoxylierten Fettalkohole und der glycosylierten Fettsäuren in einer Menge von 5-20, insbesondere 5-15 Gew.-%, und mindestens ein anionischer Emulgator aus der Gruppe der phosphorylierten Emulgatoren in einer Menge von 2-6 Gew.-% enthalten ist,
(c) mindestens ein natürliches und/oder synthetisches Öl in einer Menge von 5-25 Gew.-%, insbesondere 6-20 Gew.-%,
wobei die Mikroemulsion einen pH-Wert von 3-7 besitzt.

Die Mikroemulsion liegt vorzugsweise als Öl-in-Wasser(O/W)-Zubereitung vor. Die Mikroemulsion besitzt hierbei vorzugsweise eine kubische Phase. Die erfindungsgemäßen Mikroemulsionen können transparent, translucent oder opak sein, sind jedoch vorzugsweise transparent.

Die Viskosität der erfindungsgemäßen Zusammensetzung beträgt vorzugsweise mindestens 900 Pas bei einer Scherrate von 0,01 Hz (25°C, 1 bar), sie weist ferner vorzugsweise eine Viskositätserniedrigung bei Erwärmung von 25°C auf 70°C um mindestens 20 %, besonders bevorzugt um mindestens 50 % (1 bar) auf und/oder eine Fließgrenze von mindestens 200 Pas, besonders bevorzugt mindestens 500 Pas (1 bar) bei 25°C und 40°C. Die erfindungsgemäßen Zusammensetzungen sind vorteilhafterweise des weiteren für mindestens drei Monate bei einer Temperatur von -20°C stabil.

Eine bevorzugte Verwendung der erfindungsgemäßen Zusammensetzung ist die Verwendung als Haarbehandlungsmittel, insbesondere zum Modellieren, Formen und Stylen des Haares.

Als Applikationsort kommt entsprechend die Haut jedes Körperbereichs in Frage, insbesondere jedoch das Haar.

Der pH-Bereich der erfindungsgemäßen Zusammensetzung liegt vorzugsweise im Bereich 4,5-6,5.

### Emulgatoren

Efindungsgemäß als nichtionischen Emulgatoren verwendbar sind insbesondere Anlagerungsprodukte von Ethylenoxid an lineare Fettalkohole, an Fettsäuren, an Fettsäurealkanolamide, an Fettsäuremonoglyceride, an Sorbitan-Fettsäuremonoester, an Fettsäurealkanolamide, an Fettsäureglyceride, z.B. an gehärtetes Rizinusöl, an Methylglucosidmonofettsäureester und Gemische davon. Grundsätzlich können jedoch auch beliebige andere Emulgatoren und/oder Tenside verwendet werden.

Erfindungsgemäß verwendbare Emulgatoren in diesem Sinne sind beispielsweise
- Anlagerungsprodukte von 4 bis 250, insbesondere 4 bis 30 Mol Ethylenoxid und/oder 0 bis 5 Mol Propylenoxid an lineare C₈-C₂₂-Fettalkohole, an C₁₂-C₂₂-Fettsäuren und an C₈-C₁₅-Alkylphenole,
- C₁₂-C₂₂-Fettsäuremono- und -diester von Anlagerungsprodukten von 1 bis 250, insbesondere 1 bis 30 Mol Ethylenoxid an C₃-C₆-Polyole, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen, z. B. das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten C₈-C₂₂-Fettsäuren,
- Sterole (Sterine). Als Sterole wird eine Gruppe von Steroiden verstanden, die am C-Atom 3 des Steroid-Gerüstes eine Hydroxylgruppe tragen und sowohl aus tierischem Gewebe (Zoosterole) wie auch aus pflanzlichen Fetten (Phytosterole) isoliert werden. Beispiele für Zoosterole sind das Cholesterol und das Lanosterol. Beispiele geeigneter Phytosterole sind Beta-Sitosterol, Stigmasterol, Campesterol und Ergosterol. Auch aus Pilzen und Hefen werden Sterole, die sogenannten Mykosterole, isoliert.
- Phospholipide, vor allem die Glucose-Phospolipide, die z. B. als Lecithine bzw. Phosphatidylcholine aus z. B. Eidotter oder Pflanzensamen (z. B. Sojabohnen) gewonnen werden,
- Fettsäureester von Zuckern und Zuckeralkoholen wie Sorbit,
- Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI),
- Lineare und verzweigte C₈-C₃₀-Fettsäuren und deren Na-, K-, Ammonium-, Ca-, Mg- und Zn - Salze.

Erfindungsgemäß verwendbare nichtionische Emulgatoren sind des weiteren:
- alkoxylierte Fettsäurealkylester der Formel R¹CO-(OCH₂CHR²)ₓOR³,
   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und x für Zahlen von 1 bis 20 steht,
- Anlagerungsprodukte von Ethylenoxid an Fettsäurealkanolamide und Fettamine,
- Fettsäure-N-alkylglucamide,
- C₈ - C₂₂-Alkylamin-N-oxide,
- Alkylpolygykoside entsprechend der allgemeinen Formel RO-(Z)ₓ wobei R für eine C₈- C₁₆-Alkylgruppe, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind solche Alkylpolyglykoside, bei denen R im wesentlichen aus C₈- und C₁₀-Alkylgruppen, im wesentlichen aus C₁₂- und C₁₄-Alkylgruppen, im wesentlichen aus C₈- bis C₁₆-Alkylgruppen oder im wesentlichen aus C₁₂- bis C₁₆-Alkylgruppen besteht. Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt. Die erfindungsgemäß verwendbaren Alkylpolyglykoside enthalten im Schnitt 1,1 bis 5, bevorzugt 1,1 bis 2,0 besonders bevorzugt 1,1 bis 1,8 Zuckereinheiten. Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

5-20Gw-%, insbesonder 5-15 Gew%, nichtionischen Emulgator sind ausgewählt aus der Gruppe der ethoxylierten Fettalkohole und der glycosylierten Fettsäuren. In besonders vorteilhaften Ausführungsformen sind die nichtionischen Emulgatoren bzw. nichtionischen Tenside ausgewählt aus Oleth-5, PEG-25 hydrogeniertes Castoröl, PEG-200 hydrogeniertes Glyceryl Palmate (Antil® 200, Degussa) und Sorbitan Monolaurat sowie Mischungen davon.

Bevorzugte anionische Emulgatoren sind Alkylsulfate, Alkylpolyglycolethersulfate und Ethercarbonsäuren mit 10 bis 18 C-Atomen in der Alkylgruppe und bis zu 12 Glycolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglyceridsulfate, Alkyl- und Alkenyletherphosphate sowie Eiweißfettsäurekondensate. Zwitterionische Emulgatoren tragen im Molekül mindestens eine quartäre Ammoniumgruppe und mindestens eine -COO⁻ - oder -SO₃⁻ -Gruppe. Besonders geeignete zwitterionische Emulgatoren sind die sogenannten Betaine wie die N-Alkyl-N,N-dimethylammonium-glycinate, N-Acyl-aminopropyl-N,N-dimethylammoniumglycinate und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat.

Als anionische Emulgatoren eignen sich in erfindungsgemäßen Zusammensetzungen des weiteren alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether- und Amidgruppen sowie Hydroxylgruppen enthalten sein. Beispiele für geeignete schäumende Aniontenside sind, jeweils in Form der Natrium-, Kalium- und Ammonium- sowie der Mono-, Di- und Trialkanolammoniumsalze mit 2 bis 4 Kohlenstoffatomen in der Alkanolgruppe,
- Acylglutamate der Formel (II), 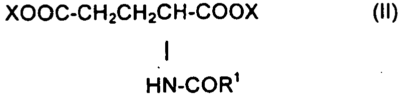 in der R'CO für einen linearen oder verzweigten Acylrest mit 6 bis 22 Kohlenstoffatomen und 0, 1, 2 oder 3 Doppelbindungen und X für Wasserstoff, ein Alkali- und/oder Erdalkalimetall, Ammonium, Alkylammonium, Alkanolammonium oder Glucammonium steht, beispielsweise Acylglutamate, die sich von Fettsäuren mit 6 bis 22, vorzugsweise 12 bis 18 Kohlenstoffatomen ableiten, wie beispielsweise C_{12/14}- bzw. C_{12/18}-Kokosfettsäure, Laurinsäure, Myristinsäure, Palmitinsäure und/oder Stearinsäure, insbesondere Natrium-N-cocoyl- und Natrium-N-stearoyl-L-glutamat,
- Ester einer hydroxysubstituierten Di- oder Tricarbonsäure der allgemeinen Formel (III), 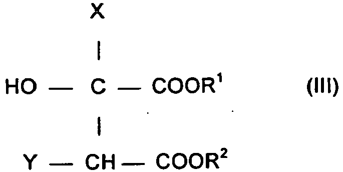 in der X=H oder eine -CH₂COOR-Gruppe ist, Y=H oder -OH ist unter der Bedingung, dass Y=H ist, wenn X=-CH₂COOR ist, R, R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten(C₆-C₁₈)-Alkylpolysaccharide mit 1 bis 6 monomeren Saccharideinheiten und/oder der veretherten aliphatischen (C₆-C₁₆)-Hydroxyalkylpolyole mit 2 bis 16 Hydroxylresten ausgewählt sind, unter der Maßgabe, daß wenigstens eine der Gruppen R, R¹ oder R² ein Rest Z ist,
- Ester des Sulfobernsteinsäure-Salzes der allgemeinen Formel (IV), 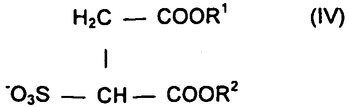 in der R¹ und R² unabhängig voneinander ein Wasserstoffatom, ein Alkali- oder Erdalkalimetallkation, eine Ammoniumgruppe, das Kation einer ammonium-organischen Base oder einen Rest Z bedeuten, der von einer polyhydroxylierten organischen Verbindung stammt, die aus der Gruppe der veretherten (C₆-C₁₈)-Alkylpolysaccharide mit 1 bis 6 monomeren Saccharideinheiten und/oder der veretherten aliphatischen(C₆-C₁₆)-Hydroxyalkylpolyole mit 2 bis 16 Hydroxylresten ausgewählt ist, unter der Maßgabe, daß wenigstens eine der Gruppen R¹ oder R² ein Rest Z ist,
- Sulfobernsteinsäuremono- und -dialkylester mit 8 bis 24 C-Atomen in der Alkylgruppe und Sulfobernsteinsäuremonoalkylpolyoxyethylester mit 8 bis 24 C-Atomen in der Alkylgruppe und 1 bis 6 Ethoxygruppen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcosinate mit einem linearen oder verzweigten Acylrest mit 6 bis 22 Kohlenstoffatomen und 0, 1, 2 oder 3 Doppelbindungen,
- Acyltaurate mit einem linearen oder verzweigten Acylrest mit 6 bis 22 Kohlenstoffatomen und 0, 1, 2 oder 3 Doppelbindungen,
- Acylisethionate mit einem linearen oder verzweigten Acylrest mit 6 bis 22 Kohlenstoffatomen und 0, 1, 2 oder 3 Doppelbindungen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)_{z}-SO₃X, in der R eine bevorzugt lineare Alkylgruppe mit 8 bis 30 C-Atomen, besonders bevorzugt mit 8 - 18 C-Atomen, z = 0 oder 1 bis 12, besonders bevorzugt 3, und X ein Natrium-, Kalium-, Magnesium-, Zink-, Ammoniumion oder ein Monoalkanol-, Dialkanol- oder Trialkanolammoniumion mit 2 bis 4 Kohlenstoffatomen in der Alkanolgruppe ist, wobei ein besonders bevorzugtes Beispiel Zinkcocoylethersulfat mit einem Ethoxylierungsgrad von z = 3 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Alkyl- und/oder Alkenyletherphosphate der Formel (V), 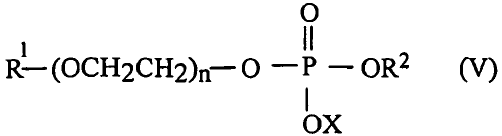 in der R' bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 1 bis 10 und X für Wasserstoff, einen Rest (CH₂CH₂O)ₙR¹, ein Alkali- oder Erdalkalimetall oder NR³R⁴R⁵R⁶, mit R³ bis R⁶ unabhängig voneinander stehend für einen C₁ bis C₄ - Kohlenwasserstoffrest, steht,
- sulfatierte Fettsäurealkylenglykolester der Formel R⁷CO(AlkO)ₙSO₃M, in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (VI), 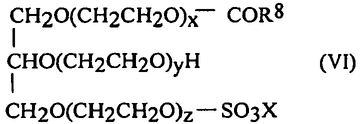 in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (VI) eingesetzt, in der R⁸CO für einen linearen Acylrest mit 8 bis 18 Kohlenstoffatomen steht.

Die in dem erfindungsgemäβen Mittel enhaltenen phosphorglierten Emulgatoren sind vorzugsweise ausgewählt aus der Gruppe der phosphorylierten alkoxylierten Emulgatoren, besonders bevorzugt aus der Gruppe der Alkyl- und/oder Alkenyletherphosphate, insbesondere derjenigen gemäß Formel (V). In vorteilhaften Ausführungsformen werden Oleth-3-phosphat, Mischungen aus Mono-, Di- und Triceteareth-4-phosphat (Hostaphat KW 340 D, Clariant) und/oder Mischungen aus Mono-, Di- und Trilaureth-4-phosphat (Hostaphat KL 340 D, Clariant) als phospholierte Emulgatoren verwendet.

Ampholytische Emulgatoren enthalten außer einer C₈ - C₂₄-Alkyl- oder -Acylgruppe mindestens eine freie Aminogruppe und mindestens eine -COOH- oder -SO₃H-Gruppe im Molekül und können innere Salze ausbilden. Beispiele für geeignete ampholytische Emulgatoren sind N-Alkylglycine, N-Alkylaminopropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe.

Als zwitterionische Emulgatoren kommen oberflächenaktive Verbindungen in Frage, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Emulgatoren sind die sogenannten Betaine wie die N-Alkyl-N,N-dimethylammoniumglycinate, beispielsweise das Kokosalkyldimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

### Fettstoffe

Die kosmetischen oder pharmazeutischen Zusammensetzungen enthalten weiterhin mindestens im natürliches und/oder synthetisches Öl.

Die Ölkomponente kann insbesondere ausgewählt sein aus
- pflanzlichen Ölen, insbesondere Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssigen Paraffinölen, Isoparaffinölen, z. B. Isohexadecan und Isoeicosan, aus hydrogenierten Polyalkenen, insbesondere Poly-1-decenen (im Handel erhätlich als Nexbase 2004, 2006 oder 2008 FG (Fortum, Belgien)), aus synthetischen Kohlenwasserstoffen, z.B. 1,3-Di-(2-ethyl-hexyl)-cyclohexan (Cetiol® S), sowie aus flüchtigen und nichtflüchtigen Siliconölen, die cyclisch, wie z. B. Decamethylcyclopentasiloxan und Dodecamethylcyclohexasiloxan, oder linear sein können, z. B. lineares Dimethylpolysiloxan, im Handel erhältlich z. B. unter der Bezeichnung Dow Corning^{®} 190, 200, 244, 245, 344, 345 oder 350 und Baysilon^{®} 350 M,
- Di-n-alkylethern mit insgesamt zwischen 12 bis 36 C-Atomen, insbesondere 12 bis 24 C-Atomen, wie beispielsweise Di-n-octylether (im Handel erhältlich als Cetiol® OE), Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether.
- Esterölen. Unter Esterölen sind zu verstehen die Ester von C₆-C₃₀-, insbesondere C₆-C₂₂-Fettsäuren, mit C₂-C₃₀-, insbesondere C₂-C₂₄-Fettalkoholen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß einsetzbar sind insbesondere Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat (Cetiol®V), Glycerintricaprylat, Kokosfettalkohol-caprinat/- caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Olsäuredecylester (Cetiol®V),
- Dicarbonsäureestern wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrischen, unsymmetrischen oder cyclischen Estern der Kohlensäure mit Fettalkoholen, beispielsweise beschrieben in der DE-OS 197 56 454, Glycerincarbonat oder Dicaprylylcarbonat (Cetiol® CC),
- Mono-, Di- und Trifettsäureestern, insbesondere Trifettsäureestern, von gesättigten und/oder ungesättigten linearen und/oder verzweigten Fettsäuren, insbesondere C₆-C₂₂-Fettsäuren, mit Glycerin, wie beispielsweise Triglyceride der Caprinsäure und/oder Caprylsäure, Monomuls® 90-018, Monomuls® 90-L12 oder Cutina® MD.

Die polare Ölkomponente kann weiterhin ausgewählt sein aus verzweigten Alkanolen, z. B. Guerbet-Alkoholen mit einer einzigen Verzweigung am Kohlenstoffatom 2 wie 2-Hexyldecanol, 2-Octyldodecanol, Isotridecanol und Isohexadecanol, aus Alkandiolen, z. B. den aus Epoxyalkanen mit 12 - 24 C-Atomen durch Ringöffnung mit Wasser erhältlichen vicinalen Diolen, aus Etheralkoholen, z. B. den Monoalkylethern des Glycerins, des Ethylenglycols, des 1,2-Propylenglycols oder des 1,2-Butandiols, aus Dialkylethern mit jeweils 12 - 24 C-Atomen, z. B. den Alkyl-methylethern oder Di-n-alkylethern mit jeweils insgesamt 12 - 24 C-Atomen, insbesondere Di-n-octylether (Cetiol^{®}OE ex Cognis), sowie aus Anlagerungsprodukten von Ethylenoxid und/oder Propylenoxid an ein- oder mehrwertige C₃₋₂₀-Alkanole wie Butanol und Glycerin, z. B. PPG-3-Myristylether (Witconol^{®} APM), PPG-14-Butylether (Ucon Fluid^{®} AP), PPG-15-Stearylether (Artamol^{®} E), PPG-9-Butylether (Breox^{®} B25) und PPG-10-Butandiol (Macol^{®} 57).

In einer erfindungsgemäß bevorzugten Ausführungsform umfasst das erfindungsgemäß eingesetzte Öl. Paraffinöl, Esteröle und Fettsäureester des Glycerins sowie Mischungen davon. Ganz besonders bevorzugt sind Paraffinöl, Isopropylmyristat, Kokosfettalkohol-caprinat/-caprylat (Coco Caprylate/Caprate) und Triglyceride der Caprinsäure und/oder Caprylsäure (Caprylic/Capric Triglycerides) sowie Mischungen davon.

Die erfindungsgemäße kosmetische oder pharmazeutische Zusammensetzung kann auch weitere Bestandteile als die zuvor genannten enthalten. In einer bevorzugten Ausführungsform enthält sie mindestens eine der im folgenden aufgezählten Substanzen. Sie kann auch jede beliebige Kombination der im folgenden aufgezählten Bestandteile enthalten.

### Hydrotrope

Zur Verbesserung des Fließverhaltens sowie gegebenenfalls zur Entfernung von Trübungen bzw. zur Sicherstellung der Transparenz der Emulsion können ferner Hydrotrope, wie beispielsweise Ethanol, Isopropylalkohol, oder Polyole eingesetzt werden. Bei den Hydrotropen handelt es sich vorzugsweise um Alkohole, die sich zu wenigstens 5 Gew.-% bei 20°C klar in Wasser lösen oder aber - im Falle langkettiger oder polymerer Alkohole - durch Erwärmen der Lösung auf 50°C bis 60°C in Lösung gebracht werden können. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind Glycerin; C₂-C₁₂-Diole, wie beispielsweise 1,6-Hexandiol, Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton; technische Oligoglyceringemische mit einem Eigenkondensationsgrad von 1,5 bis 10 wie etwa technische Diglyceringemische mit einem Diglyceringehalt von 40 bis 50 Gew.%; Methyolverbindungen, wie insbesondere Trimethylolethan, Trimethylolpropan, Trimethylolbutan, Pentaerythrit und Dipentaerythrit; Niedrigalkylglucoside, insbesondere solche mit 1 bis 8 Kohlenstoffen im Alkylrest, wie beispielsweise Methyl- und Butylglucosid; Zuckeralkohole mit 5 bis 12 Kohlenstoffatomen, wie beispielsweise Sorbit oder Mannit, Zucker mit 5 bis 12 Kohlenstoffatomen, wie beispielsweise Glucose oder Saccharose; Aminozucker, wie beispielsweise Glucamin; Dialkoholamine, wie Diethanolamin oder 2-Amino-1,3-propandiol.

Die Menge des Hydrotrops oder des Hydrotrop-Gemisches in den erfindungsgemäßen Zusammensetzungen beträgt vorzugsweise 1 - 20 Gew.-% und vorzugsweise 5 - 15 Gew.-%, bezogen auf die gesamte Zusammensetzung.

### Konservierungsmittel

Als Konservierungsmittel eignen sich beispielsweise Phenoxyethanol, Formaldehydlösung, Parabene, insbesondere Methyl- oder Propylparaben, Pentandiol oder Sorbinsäure sowie die in Anlage 6, Teil A und B der Kosmetikverordnung aufgeführten weiteren Stoffklassen. Als Insekten-Repellentien kommen N,N-Diethyl-m-toluamid, 1,2-Pentandiol oder Ethyl Butylacetylaminopropionate in Frage, als Selbstbräuner eignet sich Dihydroxyaceton.

### Hydrophil modifizierte Silicone

Die erfindungsgemäßen Zusammensetzungen können weiterhin wenigstens ein hydrophil modifiziertes Silicon enthalten. Sie ermöglichen die Formulierung hochtransparenter Zusammensetzungen, reduzieren die Klebrigkeit und hinterlassen ein frisches Hautgefühl. Unter hydrophil modifizierten Siliconen werden erfindungsgemäß Polyorganosiloxane mit hydrophilen Substituenten verstanden, welche die Wasserlöslichkeit der Silicone bedingen. Erfindungsgemäß wird unter Wasserlöslichkeit verstanden, dass sich wenigstens 2 Gew.-% des mit hydrophilen Gruppen modifizierten Silicons in Wasser bei 20 °C lösen. Entsprechende hydrophile Substituenten sind beispielsweise Hydroxy-, Polyethylenglycol- oder Polyethylenglycol/Polypropylenglycol-Seitenketten sowie ethoxylierte Ester-Seitenketten. Erfindungsgemäß bevorzugt geeignet sind hydrophil modifizierte Silicon-Copolyole, insbesondere Dimethicone-Copolyole, die beispielsweise von Wacker-Chemie unter der Bezeichnung Belsil^{®} DMC 6031, Belsil^{®} DMC 6032, Belsil^{®} DMC 6038 oder Belsil^{®} DMC 3071 VP bzw. von Dow Corning unter der Bezeichnung DC 2501 im Handel sind. Besonders bevorzugt geeignet ist die Verwendung von Belsil^{®} DMC 6038, da es die Formulierung hochtransparenter Zusammensetzungen ermöglicht, die beim Verbraucher eine höhere Akzeptanz erreichen. Erfindungsgemäß kann auch ein beliebiges Gemisch dieser Silicone eingesetzt werden.

Die Menge des hydrophil modifizierten Silicons oder des Alkohol-Gemisches in den erfindungsgemäßen Zusammensetzungen beträgt 0,5 - 10 Gew.-%, bevorzugt 1 - 8 Gew.-% und besonders bevorzugt 2 - 6 Gew.-%, bezogen auf das Gesamtgewicht der Zusammensetzung.

### Saccharide

Weiterhin können die erfindungsgemäßen Zusammensetzungen mindestens ein Mono-, Oligo- oder Polysaccharid oder deren Derivate enthalten.

Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.

Erfindungsgemäß geeignete Oligosaccharide sind aus zwei bis zehn Monosaccharideinheiten zusammengesetzt, z. B. Saccharose, Lactose oder Trehalose. Ein besonders bevorzugtes Oligosaccharid ist Saccharose. Ebenfalls besonders bevorzugt ist die Verwendung von Honig, der überwiegend Glucose und Saccharose enthält.

Erfindungsgemäß geeignete Polysaccharide sind aus mehr als zehn Monosaccharideinheiten zusammengesetzt. Bevorzugte Polysaccharide sind die aus α-D-Glucose-Einheiten aufgebauten Stärken sowie Stärkeabbauprodukte wie Amylose, Amylopektin und Dextrine. Erfindungsgemäß besonders vorteilhaft sind chemisch und/oder thermisch modifizierte Stärken, z. B. Hydroxypropylstärkephosphat, Dihydroxypropyldistärkephosphat oder die Handelsprodukte Dry Flo^{®}. Weiterhin bevorzugt sind Dextrane sowie ihre Derivate, z. B. Dextransulfat. Ebenfalls bevorzugt sind nichtionische Cellulose-Derivate, wie Methylcellulose, Hydroxypropylcellulose oder Hydroxyethylcellulose, sowie kationische Cellulose-Derivate, z. B. die Handelsprodukte Celquat^{®} und Polymer JR^{®}, und bevorzugt Celquat^{®} H 100, Celquat^{®} L 200 und Polymer JR^{®} 400 (Polyquaternium-10) sowie Polyquaternium-24. Weitere bevorzugte Beispiele sind Polysaccharide aus Fucose-Einheiten, z. B. das Handelsprodukt Fucogel^{®}. Besonders bevorzugt sind die aus Aminozuckereinheiten aufgebauten Polysaccharide, insbesondere Chitine und ihre deacetylierten Derivate, die Chitosane, und Mucopolysaccharide. Zu den erfindungsgemäß bevorzugten Mucopolysacchariden gehören Hyaluronsäure und ihre Derivate, z. B. Natriumhyaluronat oder Dimethylsilanolhyaluronat, sowie Chondroitin und seine Derivate, z. B. Chondroitinsulfat.

### Polymere

In einer vorteilhaften Ausführungsform enthalten die erfindungsgemäßen Zusammensetzungen mindestens ein filmbildendes, emulsionsstabilisierendes, verdickendes oder adhäsives Polymer, ausgewählt aus natürlichen und synthetischen Polymeren, die kationisch, anionisch, amphoter geladen oder nichtionisch sein können.

Erfindungsgemäß bevorzugt sind kationische, anionische sowie nichtionische Polymere.

Unter den kationischen Polymeren bevorzugt sind Polysiloxane mit quaternären Gruppen, z. B. die Handelsprodukte Q2-7224 (Dow Corning), Dow Corning^{®} 929 Emulsion (mit Amodimethicone), SM-2059 (General Electric), SLM-55067 (Wacker) sowie Abil^{®}-Quat 3270 und 3272 (Th. Goldschmidt).

Bevorzugte anionische Polymere, die die Wirkung des erfindungsgemäß verwendeten Wirkstoffs unterstützen können, enthalten Carboxylat- und/oder Sulfonatgruppen und als Monomere zum Beispiel Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als alleiniges Monomer oder als Comonomer 2-Acrylamido-2-methylpropansulfonsäure, wobei die Sulfonsäuregruppe ganz oder teilweise in Salzform vorliegen kann. Innerhalb dieser Ausführungsform ist es bevorzugt, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppenhaltigen Monomeren. Ein besonders bevorzugtes anionisches Copolymer besteht aus 70 bis 55 Mol-% Acrylamid und 30 bis 45 Mol-% 2-Acrylamido-2-methylpropansulfonsäure, wobei die Sulfonsäuregruppen ganz oder teilweise als Natrium-, Kalium-, Ammonium-, Mono- oder Triethanolammonium-Salz vorliegen. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Weitere bevorzugte anionische Homo- und Copolymere sind unvernetzte und vernetzte Polyacrylsäuren. Dabei können Allylether von Pentaerythrit, von Sucrose und von Propylen bevorzugte Vernetzungsagentien sein. Solche Verbindungen sind zum Beispiel die Handelsprodukte Carbopol^{®}. Ein besonders bevorzugtes anionisches Copolymer enthält als Monomer zu 80 - 98 % eine ungesättigte, gewünschtenfalls substituierte C₃₋₆-Carbonsäure oder ihr Anhydrid sowie zu 2 - 20 % gewünschtenfalls substituierte Acrylsäureester von gesättigten C₁₀₋₃₀-Carbonsäuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Geeignete nichtionische Polymere sind beispielsweise Polyvinylalkohole, die teilverseift sein können, z. B. die Handelsprodukte Mowiol^{®} sowie Vinylpyrrolidon/Vinylester-Copolymere und Polyvinylpyrrolidone, die z. B. unter dem Warenzeichen Luviskol^{®} (BASF) vertrieben werden.

### Verdicker

Erfindungsgemäß optional als Verdickungsmittel einsetzbare Verbindungen sind z. B. Celluloseether, wie Hydroxypropylcellulose, Hydroxyethylcellulose und Methylhydroxypropylcellulose, verdickende Polymere auf Basis von Polyacrylaten, die gewünschtenfalls vernetzt sein können, z. B. die Carbopoltypen oder Pemulen^{®}-Produkte, oder auf Basis von Polyacrylamiden oder sulfonsäuregruppenhaltigen Polyacrylaten, z. B Sepigel^{®} 305 oder Simulgel^{®} EG, weiterhin anorganische Verdicker, z. B. Bentonite und Hectorite (Laponite^{®}).

### Weitere Bestandteile

Die erfindungsgemäßen Zusammensetzungen können weiterhin mindestens eine α-Hydroxycarbonsäure oder α-Ketocarbonsäure oder deren Ester-, Lacton- oder Salzform enthalten. Geeignete α-Hydroxycarbonsäuren oder α-Ketocarbonsäuren sind ausgewählt aus Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Apfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren oder α-Ketocarbonsäuren oder ihre Derivate sind in Mengen von 0,1 - 10 Gew.-%, bevorzugt 0,5 - 5 Gew.-%, jeweils bezogen auf die gesamte Zusammensetzung, enthalten.

Die erfindungsgemäßen Mittel können weitere Wirk-, Hilfs- und Zusatzstoffe enthalten, beispielsweise:
- Vitamine, Provitamine und Vitaminvorstufen aus den Gruppen A, C, E und F, insbesondere 3,4-Didehydroretinol (Vitamin A₂), β-Carotin (Provitamin des Vitamin A₁), Ascorbinsäure (Vitamin C), sowie die Palmitinsäureester, Glucoside oder Phosphate der Ascorbinsäure, Tocopherole, insbesondere α-Tocopherol sowie seine Ester, z. B. das Acetat, das Nicotinat, das Phosphat und das Succinat; weiterhin Vitamin F, worunter essentielle Fettsäuren, besonders Linolsäure, Linolensäure und Arachidonsäure, verstanden werden;
- einen Ester von Retinol (Vitamin A₁) mit einer C₂₋₁₈-Carbonsäure, insbesondere Retinylacetat oder Retinylpalmitat.
- Vitamine, Provitaminen oder Vitaminvorstufen der Vitamin B-Gruppe oder deren Derivate sowie Derivate von 2-Furanon, insbesondere Vitamin B, (Thiamin), Vitamin B₂ (Riboflavin), Vitamin B₃ (Nicotinsäure und/oder Nicotinsäureamid), Vitamin B₅ (Pantothensäure und/oder Panthenol), Vitamin B₆ (Pyridoxin, Pyridoxamin und/oder Pyridoxal) und/oder Vitamin B₇ (Biotin),
- Allantoin,
- Bisabolol,
- pH-Stellmittel, insbesondere mit basischer Funktion, wie Aminomethylpropanol, insbesondere in einer Menge bis zu 2 Gew.-%,
- Antioxidantien, zum Beispiel Imidazole (z. B. Urocaninsäure) und deren Derivate, Peptide wie D,L-Carnosin, D-Carnosin, L-Carnosin und deren Derivate (z. B. Anserin), Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine,

Homocysteinsulfoximin, Butioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, das Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Katalase, Superoxid-Dismutase, Zink und dessen Derivate (z. B. ZnO, ZnSO₄), Selen und dessen Derivate (z. B. Selen-Methionin), Stilbene und deren Derivate (z. B. Stilbenoxid, trans-Stilbenoxid) und die als Antioxidans geeigneten Derivate (Salze, Ester, Ether, Zucker, Nukleotide, Nukleoside, Peptide und Lipide) dieser Wirkstoffe,
- Ceramide und Pseudoceramide,
- Triterpene, insbesondere Triterpensäuren wie Ursolsäure, Rosmarinsäure, Betulinsäure, Boswelliasäure und Bryonolsäure,
- Monomere Catechine, besonders Catechin und Epicatechin, Leukoanthocyanidine, Catechinpolymere (Catechin-Gerbstoffe) sowie Gallotannine,
- Verdickungsmittel, z. B. Gelatine, Pflanzengumme wie Agar-Agar, Guar-Gum, Alginate, Xanthan-Gum, Gummi arabicum, Karaya-Gummi oder Johannisbrotkernmehl, natürliche und synthetische Tone und Schichtsilikate, z. B. Bentonit, Hectorit, Montmorillonit oder Laponite^{®}, vollsynthetische Hydrokolloide wie z. B. Polyvinylalkohol, und außerdem Ca-, Mg- oder Zn-Seifen von Fettsäuren,
- Pflanzenglycoside,
- Strukturanten wie Maleinsäure und Milchsäure,
- Dimethylisosorbid,
- Alpha-, beta- sowie gamma-Cyclodextrine, insbesondere zur Stabilisierung von Retinol,
- Lösungsmittel, Quell- und Penetrationsstoffe wie Ethanol, Isopropanol, Ethylenglykol, Propylenglykol, Propylenglykolmonoethylether, Glycerin und Diethylenglykol, Carbonate, Hydrogencarbonate, Guanidine, Harnstoffe sowie primäre, sekundäre und tertiäre Phosphate
- Parfümöle, Pigmente sowie Farbstoffe zum Anfärben des Mittels,
- Substanzen zur Einstellung des pH-Wertes, z. B. α- und β-Hydroxycarbonsäuren,
- Komplexbildner wie EDTA, NTA, β-Alanindiessigsäure und Phosphonsäuren,
- Trübungsmittel wie Latex, Styrol/PVP- und Styrol/Acrylamid-Copolymere,
- Perlglanzmittel wie Ethylenglykolmono- und -distearat sowie PEG-3-distearat,
- Treibmittel wie Propan-Butan-Gemische, N₂O, Dimethylether, CO₂ und Luft,
- MMP-1-inhibierende Substanzen, insbesondere ausgewählt aus Photolyase und/oder T4 Endonuclease V, Propylgallat, Precocenen, 6-Hydroxy-7-methoxy-2,2-dimethyl-1(2H)-benzopyran und 3,4-Dihydro-6-hydroxy-7-methoxy-2,2-dimethyl-1(2H)-benzopyran,
- organische, mineralische und/oder modifizierte mineralische Lichtschutzfilter, insbesondere UVA-Filter und/oder UVB-Filter.

Im Rahmen einer ganz besonders bevorzugten Ausführungsform wird der nichtionische Emulgator ausgewählt aus Oleth-5, PEG-25 hydrogeniertes Castoröl, PEG-200 hydrogeniertes Glyceryl Palmate und Sorbitan Monolaurat sowie Mischungen davon, der anionische Emulgator ausgewählt aus Oleth-3-phosphat, Mischungen aus Mono-, Di- und Triceteareth-4-phosphat und/oder Mischungen aus Mono-, Di- und Trilaureth-4-phosphat, das Öl ausgewählt aus Paraffinöl, Isopropylmyristat, Kokosfettalkohol-caprinat/-caprylat (Coco Caprylate/Caprate) und Triglyceride der Caprinsäure und/oder Caprylsäure (Caprylic/Capric Triglycerides) sowie Mischungen davon.

Die folgenden Beispiele sollen die vorliegende Erfindung verdeutlichen, ohne sie hierauf zu beschränken.

### Ausführungsbeispiele

Die in den folgenden Ausführungsbeispielen gemachten Angaben sind Angaben in Gew.%, jeweils bezogen auf die Gesamtzusammensetzung.

**Beispiel 1: Basisformulierungen für transparente Mikroemulsionen**

| Beispiel | 1 | 2 | 3 | 4 | 5 | 6 | 7 | 8 | 9 |
|---|---|---|---|---|---|---|---|---|---|
| Caprylic/Capric Triglycerides | 7,5 | 7,5 | 7,5 | | | | | | |
| Isopropylmyristate | | | | 15,0 | | | | 15,0 | 15,0 |
| Cetiol LC | | | | | 15,0 | | | | |
| Cetiol V | | | | | | 15,0 | | | |
| Paraffin Oil | | | | | | | 15,0 | | |
| Arlatone GV | 9,35 | 9,35 | 12,0 | | | | | | |
| Oleth-5 | | | | 6,9 | 6,9 | 6,9 | 6,9 | 6,9 | |
| Sorbitan monolaurate | | | | | | | | | 6,9 |
| Hostaphat KW 340 D | 4,7 | 4,7 | 6,0 | | | | | | |
| Hostaphat KL 340 D | | | | | | | | 3,9 | |
| Oleth-3-phosphate | | | | 3,9 | 3,9 | 3,9 | 3,9 | | 3,9 |
| PEG-250 Distearate | 2,0 | 1,0 | 2,0 | 2,0 | 2,0 | 2,0 | 2,0 | 2,0 | 2,0 |
| Butyleneglycol | 5,0 | 5,0 | 5,0 | | | | | | |
| Sorbitol | | | | 12,5 | 12,5 | 12,5 | 12,5 | 12,5 | 12,5 |
| Antil 200 | | 3,0 | | | | | | | |
| AminomethylPropanol | | | | 0,7 | 0,7 | 0,7 | 0,7 | | 0,7 |
| Distilled Water | ad 100 | ad 100 | ad 100 | ad 100 | ad 100 | ad 100 | ad 100 | ad 100 | ad 100 |

Die folgenden Beispiele 2-5 sind weitere Beispiele für transparente Gelwachs-Mikroemulsionen. Diese Gelwachs-Mikroemulsionen waren auch nach drei Wochen Lagerung bei -20°C noch immer stabil.

### Beispiel 2:

| | |
|---|---|
| Paraffin oil | 15,00 % |
| Oleth-5 | 8,27 % |
| Oleth-3-phosphate | 4,70 % |
| PEG-150 distearate | 1,50 % |
| Sorbitol | 12,50 % |
| Butyleneglycol | 5,00 % |
| Glycerin | 33,00 % |
| Parfum | 1,0 % |
| AMP | 00,70 % |
| Distilled water | ad 100 % |

Die Ölphase wird auf 80°C erwärmt und dann zur Wasserphase hinzugegeben.

Die Mischung wird danach unter Rühren abgekühlt. Anschließend werden ggf. empfindliche Wirkstoffe oder Parfüm hinzugegeben. Man erhält eine klare O/W-Mikroemulsion mit einer Viskosität von 10000 Pas bei einer Scherrate von 0,01 Hz (25°C, 1 bar) und einer Fließgrenze bei 20°C von 1000 Pa.

### Beispiel 3:

| | |
|---|---|
| Isopropylmyristate | 15,00 % |
| Oleth-5 | 6.90 % |
| Oleth-3-phosphate | 3,90 % |
| PEG-250 distearate | 2,0 % |
| Sorbitol | 12,50 % |
| Butyleneglycol | 5,00 % |
| Glycerin | 3,00 % |
| Parfum | 1,0 % |
| AMP | 0,7 % |
| Distilled water | ad 100 % |

Die Ölphase wird auf 80°C erwärmt und dann zur Wasserphase hinzugegeben.

Die Mischung wird danach unter Rühren abgekühlt. Anschließend werden ggf. empfindliche Wirkstoffe oder Parfüm hinzugegeben. Man erhält eine klare O/W-Mikroemulsion mit einer Viskosität von 1000 Pas bei einer Scherrate von 0,01 Hz (25°C, 1 bar) und einer Fließgrenze bei 20°C von 1000 Pa.

### Beispiel 4:

| | |
|---|---|
| Hostaphat KW 340 D | 4,7 % |
| Arlatone GV | 9,35 % |
| PEG-150 Distearate | 2,0 % |
| Antil 200 | 4,0 % |
| Butyleneglycol | 5,0 % |
| Caprylic/Capric Triglycerides | 7,5 % |
| Parfum | 1,5 % |
| Distilled water | ad 100 % |

Die Ölphase wird auf 80°C erwärmt und dann zur Wasserphase hinzugegeben.

Die Mischung wird danach unter Rühren abgekühlt. Anschließend werden ggf. empfindliche Wirkstoffe oder Parfüm hinzugegeben. Man erhält eine klare O/W-Mikroemulsion mit einer Viskosität von 1000 Pas bei einer Scherrate von 0,01 Hz (25°C, 1 bar) und einer Fließgrenze bei 20°C von 300 Pa.

### Beispiel 5:

| | |
|---|---|
| Hostaphat KW 340 D | 4,7 % |
| Arlatone GV | 9,35 % |
| PEG-250 Distearate | 2,0 % |
| Antil 200 | 4,0 % |
| Butyleneglycol | 5,0 % |
| Caprylic/Capric Triglycerides | 7,5 % |
| Parfum | 1,5 % |
| Distilled water | ad 100 % |

Die Ölphase wird auf 80°C erwärmt und dann zur Wasserphase hinzugegeben.

Die Mischung wird danach unter Rühren abgekühlt. Anschließend werden ggf. empfindliche Wirkstoffe oder Parfüm hinzugegeben. Man erhält eine klare O/W-Mikroemulsion mit einer Viskosität von 4000 Pas bei einer Scherrate von 0,01 Hz (25°C, 1 bar) und einer Fließgrenze bei 20°C von 700 Pa.

### Beispiel 6:

| | |
|---|---|
| Cetiol LC | 15,00 % |
| Oleth-5 | 8,27 % |
| Oleth-3-phosphate | 4,70 % |
| PEG-150 distearate | 1,50 % |
| Sorbitol | 12,50 % |
| Butyleneglycol | 5,00 % |
| Glycerin | 3,00 % |
| Parfum | 1,0 % |
| AMP | 0,7 % |
| Distilled water | ad 100 % |

Die Ölphase wird auf 80°C erwärmt und dann zur Wasserphase hinzugegeben.

Die Mischung wird danach unter Rühren abgekühlt. Anschließend werden ggf. empfindliche Wirkstoffe oder Parfüm hinzugegeben. Man erhält eine klare O/W-Mikroemulsion mit einer Viskosität von 8000 Pas bei einer Scherrate von 0,01 Hz (25°C, 1 bar).

| | |
|---|---|
| Übersicht über die verwendeten Handelsprodukte | |
| INCl | Handelsname |
| Oleth-5 | Eumulgin 05 |
| Oleth-3-phosphate | Crodafos N3A |
| PEG-250 Distearate | Emanon 3299 R |
| PEG-150 Distearate | Aculyn 60 |
| PEG-14 Dimethicone | Abil B 8843 |
| Paraffin oil | Paraffin oil DAB 9, 35 cp |
| Butylene glycol | Butylene glycol |
| Sorbitol | Sorbit 70 % DAB |
| Glycerin | Glycerin DAB 9 |

## Patentansprüche

1. Kosmetische oder pharmazeutische Zusammensetzung in Form einer Mikroemulsion, enthaltend
(a) mindestens einen Diester aus PEG 100-500 und einer C₁₀₋₂₆-Carbonsäure in einer Menge von 0,5-4 Gew.-%, insbesondere 1-3 Gew.-%, als Molekül, das eine hydrophile Kette umfasst, die beidseitig hydrophobe Köpfe aufweist,
(b) mindestens einen Emulgator in einer Menge von 5-25 Gew.-%, wobei mindestens ein nichtionischer Emulgator ausgewählt aus der Gruppe der ethoxylierten Fettalkohole und der glycosylierten Fettsäuren in einer Menge von 5-20, insbesondere 5-15 Gew.-%, und mindestens ein anionischer Emulgator aus der Gruppe der phosphorylierten Emulgatoren in einer Menge von 2-6 Gew.-% enthalten ist,
(c) mindestens ein natürliches und/oder synthetisches Öl in einer Menge von 5-25 Gew.-%, insbesondere 6-20 Gew.-%,
wobei die Mikroemulsion einen pH-Wert von 3-7 besitzt.

2. Kosmetische oder pharmazeutische Zusammensetzung nach Anspruch 1, **dadurch gekennzeichnet, dass** es sich um eine O/W-Mikroemulsion handelt.

3. Kosmetische oder pharmazeutische Zusammensetzung nach Anspruch 1 oder 2 **dadurch gekennzeichnet, dass** der anionische Emulgator ausgewählt ist aus der Gruppe der Alkyl- und/oder Alkenyletherphosphate.

4. Kosmetische oder pharmazeutische Zusammensetzung nach einem der Ansprüche 1 bis 3, **dadurch gekennzeichnet, dass** das Öl ausgewählt ist aus der Gruppe bestehend aus Paraffinöl, Esteröle und Fettsäureester des Glycerins sowie Mischungen davon.

5. Kosmetische oder pharmazeutische Zusammensetzung nach Anspruch 4, **dadurch gekennzeichnet, dass** das Öl ausgewählt ist aus Paraffinöl, Isopropylmyristat, Kokosfettalkoholcaprinat/-caprylat und Triglyceriden der Caprinsäure und/oder Caprylsäure sowie Mischungen davon.

6. Kosmetische oder pharmazeutische Zusammensetzung nach einem der Ansprüche 1 bis 5, **dadurch gekennzeichnet, dass** die Zusammensetzung eine Viskosität von mindestens 900 Pas bei einer Scherrate von 0,01 Hz (25°C, 1 bar) besitzt.

7. Kosmetische oder pharmazeutische Zusammensetzung nach einem der Ansprüche 1 bis 6, **dadurch gekennzeichnet, dass** die Zusammensetzung eine Viskositätserniedrigung bei Erwärmung von 25°C auf 70°C um mindestens 20 % (1 bar) aufweist.

8. Kosmetische oder pharmazeutische Zusammensetzung nach einem der Ansprüche 1 bis 7, **dadurch gekennzeichnet, dass** die Zusammensetzung eine Fließgrenze bei 25°C und 40°C von mindestens 200 Pas (1 bar) besitzt.

9. Kosmetische oder pharmazeutische Zusammensetzung nach einem der Ansprüche 1 bis 8, **dadurch gekennzeichnet, dass** es sich um eine transparente Zubereitung handelt.

10. Kosmetische oder pharmazeutische Zusammensetzung nach einem der Ansprüche 1 bis 9, **dadurch gekennzeichnet, dass** sie kubische Phasen aufweist.

11. Kosmetische oder pharmazeutische Zusammensetzung nach einem der Ansprüche 1 bis 10, **dadurch gekennzeichnet, dass** es sich um ein Haarbehandlungsmittel handelt.

12. Kosmetische oder pharmazeutische Zusammensetzung nach Anspruch 11, **dadurch gekennzeichnet, dass** es es sich um ein Haarwachs oder Gelwachs handelt.

13. Verwendung einer Zusammensetzung nach einem der Ansprüche 1 bis 12 zum Modellieren und/oder Formen einer Frisur.

14. Verwendung einer Zusammensetzung nach einem der Ansprüche 1 bis 12 zum Stylen des Haares.

## Claims

1. A cosmetic or pharmaceutical composition in the form of a microemulsion, containing
(a) at least one diester prepared from PEG 100-500 and a C₁₀₋₂₆ carboxylic acid in a quantity of 0.5-4 wt.%, in particular of 1-3 wt.%, as a molecule which comprises a hydrophilic chain which has hydrophobic heads on both ends,
(b) at least one emulsifier in a quantity of 5-25 wt.%, wherein at least one nonionic emulsifier selected from the group of ethoxylated fatty alcohols and glycosylated fatty acids is present in a quantity of 5-20, in particular of 5-15 wt.%, and at least one anionic emulsifier from the group of phosphorylated emulsifiers is present in a quantity of 2-6 wt.%,
(c) at least one natural and/or synthetic oil in a quantity of 5-25 wt.%, in particular of 6-20 wt.%,
wherein the microemulsion has a pH value of 3-7.

2. A cosmetic or pharmaceutical composition according to claim 1, **characterised in that** it is an O/W microemulsion.

3. A cosmetic or pharmaceutical composition according to claim 1 or claim 2, **characterised in that** the anionic emulsifier is selected from the group of alkyl and/or alkenyl ether phosphates.

4. A cosmetic or pharmaceutical composition according to any one of claims 1 to 3, **characterised in that** the oil is selected from the group consisting of paraffin oil, ester oils and fatty acid esters of glycerol and mixtures thereof.

5. A cosmetic or pharmaceutical composition according to claim 4, **characterised in that** the oil is selected from paraffin oil, isopropyl myristate, coconut fatty alcohol caprate/caprylate and triglycerides of capric acid and/or caprylic acid and mixtures thereof.

6. A cosmetic or pharmaceutical composition according to any one of claims 1 to 5, **characterised in that** the composition has a viscosity of at least 900 Pa·s at a shear rate of 0.01 Hz (25°C, 1 bar).

7. A cosmetic or pharmaceutical composition according to any one of claims 1 to 6, **characterised in that** the composition undergoes a reduction in viscosity of at least 20% (1 bar) when heated from 25°C to 70°C.

8. A cosmetic or pharmaceutical composition according to any one of claims 1 to 7, **characterised in that** the composition has a flow limit at 25°C and 40°C of at least 200 Pa·s (1 bar).

9. A cosmetic or pharmaceutical composition according to any one of claims 1 to 8, **characterised in that** it is a transparent preparation.

10. A cosmetic or pharmaceutical composition according to any one of claims 1 to 9, **characterised in that** it comprises cubic phases.

11. A cosmetic or pharmaceutical composition according to any one of claims 1 to 10, **characterised in that** it is a hair treatment agent.

12. A cosmetic or pharmaceutical composition according to claim 11, **characterised in that** it is a hair wax or gel wax.

13. Use of a composition according to any one of claims 1 to 12 for modelling and/or shaping a hairstyle.

14. Use of a composition according to any one of claims 1 to 12 for styling hair.

## Revendications

1. Composition cosmétique ou pharmaceutique sous la forme d'une microémulsion, contenant
a) au moins un diester à base de PEG 100-500 et d'un acide carboxylique en C₁₀-C₂₆ en une quantité de 0,5 à 4 % en poids, en particulier de 1 à 3 % en poids, à titre de molécule qui comprend une chaîne hydrophile qui présente des têtes hydrophobes des deux côtés ;
b) au moins un émulsifiant en une quantité de 5-25 % en poids, contenant au moins un émulsifiant non ionique choisi parmi le groupe des alcools gras éthoxylés et des alcools gras glycosylés, en une quantité de 5 à 20, en particulier de 5 à 15 % en poids, et au moins un émulsifiant anionique choisi parmi le groupe des émulsifiants phosphorylés, en une quantité de 2 à 6 % en poids ;
c) au moins une huile naturelle et/ou synthétique en une quantité de 5 à 25 % en poids, en particulier de 6 à 20 % en poids ;
la microémulsion possédant une valeur de pH de 3 à 7.

2. Composition cosmétique ou pharmaceutique selon la revendication 1, **caractérisée en ce qu'**il s'agit d'une microémulsion du type huile dans l'eau.

3. Composition cosmétique ou pharmaceutique selon la revendication 1 ou 2, **caractérisée en ce que** l'émulsifiant anionique est choisi parmi le groupe des alkyl- et/ou alcényl-étherphosphates.

4. Composition cosmétique ou pharmaceutique selon l'une quelconque des revendications 1 à 3, **caractérisée en ce que** l'huile est choisie parmi le groupe constitué par l'huile de paraffine, des huiles d'esters et des esters d'acides gras du glycérol, ainsi que leurs mélanges.

5. Composition cosmétique ou pharmaceutique selon la revendication 4, **caractérisée en ce que** l'huile est choisie parmi le groupe constitué par l'huile de paraffine, le myristate d'isopropyle, le caprate/caprylate d'alcool gras de coco, et des triglycérides de l'acide caproïque et/ou de l'acide caprylique, ainsi que leurs mélanges.

6. Composition cosmétique ou pharmaceutique selon l'une quelconque des revendications 1 à 5, **caractérisée en ce que** la composition possède une viscosité d'au moins 900 Pas à un taux de cisaillement de 0,01 Hz (25 °C, 1 bar).

7. Composition cosmétique ou pharmaceutique selon l'une quelconque des revendications 1 à 6, **caractérisée en ce que** la composition présente une diminution de la viscosité lors d'un réchauffement de 25 °C à 70 °C d'au minimum 20 % (1 bar).

8. Composition cosmétique ou pharmaceutique selon l'une quelconque des revendications 1 à 7, **caractérisée en ce que** la composition possède une limite d'écoulement à 25 °C et à 40 °C d'au moins 200 Pas (1 bar).

9. Composition cosmétique ou pharmaceutique selon l'une quelconque des revendications 1 à 8, **caractérisée en ce qu'**il s'agit d'une préparation transparente.

10. Composition cosmétique ou pharmaceutique selon l'une quelconque des revendications 1 à 9, **caractérisée en ce qu'**elle présente des phases cubiques.

11. Composition cosmétique ou pharmaceutique selon l'une quelconque des revendications 1 à 10, **caractérisée en ce qu'**il s'agit d'un agent de traitement capillaire.

12. Composition cosmétique ou pharmaceutique selon la revendication 11, **caractérisée en ce qu'**il s'agit d'une cire capillaire ou d'une cire sous forme de gel.

13. Utilisation d'une composition selon l'une quelconque des revendications 1 à 12, pour le modelage et/ou la mise en forme d'une coiffure.

14. Utilisation d'une composition selon l'une quelconque des revendications 1 à 12, pour donner un style particulier aux cheveux.
